Europäisches Patentamt

European Patent Office

Office européen des brevets

(19)

(11)   **EP 1 341 890 B1**

(12)   **EUROPEAN PATENT SPECIFICATION**

(45) Date of publication and mention
of the grant of the patent:
**29.11.2006  Bulletin 2006/48**

(21) Application number: **01989585.3**

(22) Date of filing: **03.12.2001**

(51) Int Cl.:
***C11D 3/37*** (2006.01)

(86) International application number:
**PCT/EP2001/014382**

(87) International publication number:
**WO 2002/048305 (20.06.2002 Gazette 2002/25)**

(54) **IMPROVEMENTS RELATING TO FABRIC CARE**

VERBESSERUNGEN BEI DER TEXTILPFLEGE

AMELIORATIONS CONCERNANT L'ENTRETIEN DES TISSUS

(84) Designated Contracting States:
**AT BE CH CY DE DK ES FI FR GB GR IE IT LI LU MC NL PT SE TR**

(30) Priority: **11.12.2000  GB 0030177
11.04.2001  GB 0109142**

(43) Date of publication of application:
**10.09.2003  Bulletin 2003/37**

(73) Proprietors:
• **UNILEVER PLC
London EC4P 4BQ (GB)**
Designated Contracting States:
**CY GB IE**
• **UNILEVER N.V.
3013 AL  Rotterdam (NL)**
Designated Contracting States:
**AT BE CH DE DK ES FI FR GR IT LI LU MC NL PT SE TR**

(72) Inventors:
• **FELTON, Julie,
Unilever Research Port Sunlight
Wirral,
Merseyside CH63 3JW (GB)**
• **JOHNSON, Amanda,
Unilever Research Port Sunlight
Wirral,
Merseyside CH63 3JW (GB)**
• **JONES, Christopher Clarkson,
Unilever Research
Wirral,
Merseyside CH63 3JW (GB)**
• **OAKES, John,
Unilever Research Port Sunlight
Wirral,
Merseyside CH63 3JW (GB)**

(74) Representative: **Elliott, Peter William et al
Unilever Patent Group
Colworth House
Sharnbrook
Bedford, MK44 1LQ (GB)**

(56) References cited:
**WO-A-00/24853          WO-A-01/53600
WO-A-01/59053          WO-A-97/28239
WO-A-99/41347          US-A- 5 827 813**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

**Technical Field**

The present invention is concerned with improvements relating to fabric care and in particular to means by which the apparent ageing of clothes can be reduced or retarded.

**Background of the Invention**

It is well known that clothes, particularly lower cost and quality clothes, lose dye when washed. Almost everyone who washes a mix of clothes has at some time experienced the detrimental effects of washing a new dyed article with white articles. In simple terms, the dye is transferred from the dyed article to the white articles causing a significant change in their colour. The commonplace solution to this problem is to wash new clothes separately.

Articles which are not new also release dye, but at a reduced rate. For this reason it has been proposed to incorporate various 'dye fixatives', 'dye scavengers' or 'colour-safe' bleaches for dyestuffs in laundry compositions. These have been the subject of intensive and extensive research and variously fix the dye in place, prevent it re-depositing or chemically bleach it so as to overcome the problem of dye transfer in mixed washes.

It is well established that relatively small amounts of dye are released from older garments and that the bulk of dye release occurs in the first wash of a new garment. As will be explained in further detail below, this initial dye loss is seldom noted by users except in incidents of dye transfer.

WO 01/53600 (P&G: 2000) relates to fabric dye protection which is specific to denim. As noted in that specification and otherwise known, denim is a rather unique cloth. First, it is woven from two forms of yarn, one of which is dyed and the other is not. Second, the dyed yarn is typically dyed with indigo and has most of the dye located in the outer fibres of the yarn. This form of dyeing is known as 'ring-dyeing' and results in a fabric where the dye is easily removed by abrasion during wear: a process known in the art as 'crocking'. Thus, during wear, the dye becomes detached from the fabric, particularly on the seat, knees and thighs and is then easily extracted during washing.

The characteristic colour loss from denim is thought by some to be a desirable feature. Some may even go so far as to deliberately augment damage to denim goods to make the goods appear 'older'. Others, (according to the applicants of WO 01/53600) see such colour loss as a problem.

WO 01/53600 suggests that this rather specific problem can be overcome by the combined uses of a cationic polymer, a low molecular weight polyamine, crystal growth inhibitor and dye fixing agent to provide protection to denim fabric from dye loss which is primarily due to mechanical loss. This mechanical loss occurs through the normal abrasive destruction of the fabric during wear. The agents proposed to overcome this, apparently bind to the cloth and fix the colour in place and thereby prevent or reduce the appearance of ageing.

The characteristic colour loss of denim, which arises due to its peculiar structure, is somewhat unique. Very few other garments are purposefully ring-dyed so as to encourage colour loss.

Ageing in garments which have not been ring-dyed still presents problems.

As noted above, a large part if not the bulk of, colour loss occurs in the first wash. Despite this, clothes are not generally perceived as 'old' after the first wash. Indeed, the first wash of an article generally produces no appreciable change in appearance to the normal observer. While there may have been a significant change in hue or colour depth, this is simply not perceived without a suitable comparison. Thus, prevention of actual colour loss (by the use of dye transfer inhibitors, dye fixatives etc) is not in itself sufficient to prevent apparent ageing of garments.

WO 97/28239 relates to rinse-added fabric softening compositions which comprise an insoluble, i.e. dispersible, polyethylene in the form of an emulsion and a cationic surfactant. It is believed that the cationic surfactant adsorbs onto the surface of the polyethylene emulsion droplets and lends them a positive charge to promote adsorption of the material onto the articles being washed. In the alternative, it is possible to use cationically modified polyethylene which can adsorb directly. The materials are thus being adsorbed onto the cloth during the rinse by virtue of the cationic charges which they have or which they are associated with.

**Brief Description of the Invention**

We have determined that the most significant cue for ageing in clothes and other articles is colour loss which is localised rather than general. While an article may lose a significant proportion of its colour overall, this loss is not perceived by the casual spectator unless it is extreme. Whereas any localised colour loss, for example on seams, acts as a strong indication that the article is 'faded'.

In addition, we have determined that actual removal of dyestuff is not required for an apparent colour loss to be perceived. On seams and the like, abrasion of the surface is sufficient to produce a modified scattering of light which gives the appearance of loss of dyestuff. In reality, the dyestuff may still be present.

Moreover, we have determined that colour loss during laundering, as opposed to during wear, is a significant source of colour loss.

We have determined that this problem may be overcome by use of an anionic or nonionic polymeric lubricant which does not adsorb onto textiles in a main wash composition, during the laundering process, to prevent the visible appearance of local colour loss through mechanical damage by the laundering process.

By use of a lubricant in this manner, it is believed that localised abrasion of the articles being washed may be reduced or retarded.

Without wishing to be limited by reference to any theory of operation, it is believed that, when dyed textiles fade, this is, almost without exception, characterised by an increase in the luminance component of the colour. It is known that human ability to perceive small differences in lightness is spatially dependent [M.D. Fairchild, 'Colour Appearance models', Addison Wesley Longman Publishing Co, New York (1998)]. The maximum in sensitivity corresponds to a spatial frequency in the range 2-15 cycles per degree. It just so happens that when a garment (such as a pair of jeans) is viewed at a typical viewing distance, then the laundry-induced faded features tend to fall within this range of spatial frequencies. Thus, relatively small changes in colour can strongly influence the perception of the garment provided that they are localised.

While generalised colour loss may still occur to a significant and easily measurable extent, the perceived effect of this colour loss is greatly reduced when the colour loss is even. The effect of preventing local colour loss is to significantly reduce the degree to which the articles being washed appear visibly aged.

## Definition of the Invention

According to the invention, there is provided a use of an anionic or nonionic polymeric lubricant, which does not adsorb onto textiles, in a main wash composition, during the laundering process, to prevent the visible appearance of local colour loss through mechanical damage by the laundering process.

## Detailed Description of the Invention

The extent of mechanical damage to articles being laundered will vary with the machine type, conditions and the stage of the wash being considered. Use of low water levels and violent agitation are envisaged to promote damage whereas higher water levels and less violent agitation will reduce damage. Unfortunately, mechanised washing machines and environmental concerns have led to increased agitation and lower water levels.

The degree of agitation and the level of water present vary during the laundry cycle and it is preferable that the lubricant is present in those parts of the cycle which involve low water and/or high agitation.

The lubricants are polymeric materials.

The in-wash protection is achieved by the use of a material which does not adsorb onto the fabrics being washed. It is believed that this avoids adverse effects on the desirable properties of the fabric once it is in the post-wash dry state. For example there is preferably no stiffening of the fabric, no increased tendency for the fabric to attract and retain soils or stains, no reduction in the breathability or water absorbency characteristics of the fabric.

The lubricants are species which do not carry a cationic charge. It is believed that anionic materials exhibit better performance due to their tendency to form an extended structure.

In order that the invention may be further understood it will be described hereinafter with reference to various preferred features.

## Lubricants:

It is possible to define suitable lubricants in several ways.

Preferred lubricants show a reduction of friction on wet cotton. Preferred lubricants in the context of this invention are materials which, when measured using the frictional technique described below and with a solution containing 1 g $dm^{-3}$ of the material, give an $I_{wcl}$ (index of wet cotton lubrication) greater than 15. Preferably this index is greater than 50 more preferably greater than 75.

Preferred lubricants exhibit particular viscosity properties. A preferred lubricant in the context of this invention is a material which when dissolved at a concentration of 1 g $dm^{-3}$ in water at 25°C gives a solution that has a viscosity greater than 0.05 Pa s when measured at a shear rate of 100 $s^{-1}$.

Suitable lubricants include:

- polyacrylate salts with mol wt greater than 100 000 Dalton, preferably greater than 500,000 Dalton;

- polyacrylic acids with mol wt greater than 100,000 Dalton, preferably greater than 500,000 Dalton;

- polyacrylamides with mol wt greater than 100,000 Dalton preferably greater than 500,000 Dalton; or,

- co-polymers of these various acrylic materials.

Also suitable are dextrans, preferably with mol wt greater than 50,000 Dalton and preferably greater than 200,000 Dalton.

Other suitable materials are poly vinyl pyrrolidones with mol wt greater than 100,000 Dalton; polydimethyl siloxanes emulsified in nonionic surfactant; and, dispersions of lightly oxidised polyethylene wax e.g. (Imacol C™ ex Clariant).

It is preferable that the lubricant material is presented together with a textile-compatible carrier. In general, it is preferred that the carrier makes up the bulk of a product for use according to the present invention.

Levels for the polymeric lubricants in compositions for use according to the invention fall in the range 0.05%wt-20%wt, with levels of 0.5-5%wt being preferred. Unless otherwise stated all % values given in this specification are %wt and all ratios are weight ratios.

As these levels of lubricants are relatively low, powders, granules, tablets, liquids and other forms of detergent product can be manufactured by conventional means and the lubricants included in the formulations as appropriate.

## Surfactants:

The main wash composition of the present invention may comprise a textile-compatible carrier which may be chosen from soap and non-soap anionic, cationic, nonionic, amphoteric and zwitterionic detergent active compounds, and mixtures thereof.

Many suitable detergent active compounds are available and are fully described in the literature, for example, in "Surface-Active Agents and Detergents", Volumes I and II, by Schwartz, Perry and Berch.

The preferred textile-compatible carriers that can be used are soaps and synthetic non-soap anionic and nonionic surfactant compounds.

Anionic surfactants are well-known to those skilled in the art. Commonly employed materials include alkylbenzene sulphonates, particularly linear alkylbenzene sulphonates having an alkyl chain length of $C_8$-$C_{15}$; primary and secondary alkyl sulphates, particularly $C_8$-$C_{15}$ primary alkyl sulphates; alkyl ether sulphates; olefin sulphonates; alkyl xylene sulphonates; dialkyl sulphosuccinates; and fatty acid ester sulphonates. Sodium salts are generally preferred.

Nonionic surfactants that may be used include the primary and secondary alcohol ethoxylates, especially the $C_8$-$C_{20}$ aliphatic alcohols ethoxylated with an average of from 1 to 20 moles of ethylene oxide per mole of alcohol, and more especially the $C_{10}$-$C_{15}$ primary and secondary aliphatic alcohols ethoxylated with an average of from 1 to 10 moles of ethylene oxide per mole of alcohol. Non-ethoxylated nonionic surfactants include alkylpolyglycosides, glycerol monoethers, and polyhydroxyamides (glucamide).

Cationic surfactants that may be used include quaternary ammonium salts of the general formula $R_1R_2R_3R_4N^+$ $X^-$ wherein the R groups are independently hydrocarbyl chains of $C_1$-$C_{22}$ length, typically alkyl, hydroxyalkyl or ethoxylated alkyl groups, and X is a solubilising anion (for example, compounds in which $R_1$ is a $C_8$-$C_{22}$ alkyl group, preferably a $C_8$-$C_{10}$ or $C_{12}$-$C_{14}$ alkyl group, $R_2$ is a methyl group, and $R_3$ and $R_4$, which may be the same or different, are methyl or hydroxyethyl groups); and cationic esters (for example, choline esters) and pyridinium salts.

The total quantity of detergent surfactant in the composition is suitably from 0.1 to 60 wt%, preferably 0.5-55 wt%, more preferably 5-50 wt%.

Preferably, the quantity of anionic surfactant (when present) is in the range of from 1 to 50% by weight of the total composition. More preferably, the quantity of anionic surfactant is in the range of from 3 to 55% by weight, e.g. 5 to 30% by weight.

Preferably, the quantity of nonionic surfactant when present is in the range of from 2 to 25% by weight, more preferably from 5 to 20% by weight.

Amphoteric surfactants may also be used, for example amine oxides or betaines.

## Detergency Builders:

The compositions may suitably contain from 10 to 70%, preferably from 15 to 70% by weight, of detergency builder. Preferably, the quantity of builder is in the range of from 15 to 50% by weight.

The detergent composition may contain as builder a crystalline aluminosilicate, preferably an alkali metal alumino-silicate, more preferably a sodium alumino-silicate.

The alumino-silicate may generally be incorporated in amounts of from 10 to 70% by weight (anhydrous basis), preferably from 25 to 50%. Alumino-silicates are materials having the general formula:

$$0.8\text{-}1.5 \ M_2O \ Al_2O_3. \ 0.8\text{-}6 \ SiO_2$$

where M is a monovalent cation, preferably sodium. These materials contain some bound water and are required to have a calcium ion exchange capacity of at least 50 mg CaO/g. The preferred sodium alumino-silicates contain 1.5-3.5 $SiO_2$ units in the formula above. They can be prepared readily by reaction between sodium silicate and sodium aluminate, as amply described in the literature.

**Minor and other components:**

Other optional and minor ingredients include emulsifiers, electrolytes (for example, sodium chloride or calcium chloride) preferably in the range from 0.01 to 5% by weight, pH buffering agents, and perfumes (preferably from 0.1 to 5% by weight).

Further optional ingredients include one or more of nonaqueous solvent, perfume carriers, fluorescers; colourants, hydrotropes, antifoaming agents, enzymes, optical brightening agents, opacifiers, anti-shrinking agents, anti-wrinkle agents, anti-spotting agents, germicides, fungicides, anti-oxidants, UV absorbers (sunscreens), heavy metal sequestrants, chlorine scavengers, dye fixatives, anti-corrosion agents, drape imparting agents, antistatic agents and ironing aids. This list is not intended to be exhaustive.

**Product Form and Presentation:**

The compositions of the invention may be in the form of a liquid, solid (e.g. powder or tablet), a gel or paste, spray, stick or a foam or mousse. The composition may also be applied to a substrate (e.g. a flexible sheet) or used in a dispenser which can be used in the wash cycle.

The invention takes the form of incorporation and use of lubricants in main wash products. In such compositions the product form is generally a powder or other particulate form ranging in size from a granulate to a tablet, or a liquid. Liquids according to the present invention may be structured or isotropic.

The fabrics which treated according to the present invention are generally in the form of garments and preferably comprise cellulosic fibres, preferably from 1% to 100% cellulosic fibres (more preferably 5% to 100% cellulosic fibres, most preferably 40% to 100% such as 75% to 100%). When the fabric contains less than 100% cellulosic fibres, the balance comprises other fibres or blends of fibres suitable for use in garments such as polyester or polyamide, for example. Preferably, the cellulosic fibres are of cotton or regenerated cellulose such as viscose.

It is particularly preferred that the composition will be packaged together with instructions for use in a main wash, in an automatic washing machine.

In order that the invention may be further understood it will be described hereinafter with reference to the following non-limiting examples.

**Examples**

**Example 1 - Eye-Tracking studies:**

A pair of black cotton jeans were subjected to 10 repeat wash and dry cycles using the 40' C cotton cycle in a Miele™ front loading automatic washing machine and a 1 hour dry cycle in a Miele™ tumble drier. The jeans were not worn between washes.

These conditions generated a typical pattern of in-wash induced uneven colour fading: specifically a periodic light dark pattern along the double seam of the leg and increased colour fading at the seams of the pocket, fly and waistband. Colour matched digital images were produced of the front and back of the jeans. These images were used as stimulus material in the following experiment to determine how a human observer perceives that a garment has faded. The subject was seated in front of a computer monitor screen. Below the screen was position a small device which uses an infra red tracking system that, once calibrated, is able to determine in which direction the subject is looking. This can then be transformed to calculate which part of the on-screen image the subject is fixating. This information is recorded for the duration of the experiment so that it is possible to reconstruct the subject's eye movements and, in particular, determine the sequence, location and duration of their fixation at different parts of the on-screen image.

After initial calibration, the subject was informed of their task. This was to look at the on screen image and determine which of the two garments shown had been washed the greater number of times. Initially, they were presented with a blank screen apart from a coloured circle, the fixation point, which they were told to look at. This was then replaced with the image of the front and back of the same pair of jeans. From this point, the subject's eye movements were recorded until they voiced their decision. Two images of the same garment were used to ensure that differences were not sufficiently large that the observers could decide instantly using peripheral vision alone. The experiment was repeated using six different subjects.

The recorded eye-tracks showed that without exception, the great majority of fixations and fixation time was associated with the features of the garment showing uneven rather than even colour fading namely: the seams, fly, pockets and waistband.

## Example 2 - Human Sensitivity to Colour Changes:

The following experiment was performed to quantify the enhanced sensitivity of human colour change perception to uneven colour change compared to uniform colour change at a spatial frequency corresponding to abrasion induced faded features on washed garments.

Two sets of calibrated colour chips were produced using Adobe Photoshop™ in a calibrated printing set-up. One set of chips consisted of a number of pairs of chips of uniform colour where the colour differed only in its lightness value. The differences covered a range of values. Six series of chips of different hue were used. The second set of chips consisted of the same series except that this time the lighter of the two chips had the light colour superimposed as a band down the centre of the chip on top of the original colour. The width of the band was such that, at the viewing distance, the spatial frequency was 2 cycles per degree. 24 panellists were asked to put each of the series in the two sets into order of increased fading. Their performance was analysed using a Probit™ Statistical Analysis which then gave the 'just noticeable difference' (JND) value for colour fading perception.

The results are tabulated below (Table 1) in terms of CIELAB Delta-E values. (Delta-E represents the magnitude of colour difference). It is clear that while the absolute magnitudes vary with hue, there is a consistent ability of the panellist to discriminate lower levels of colour fading when presented with an unevenly faded sample compared to a uniformly faded sample.

**Table 1:**

| 'Just noticeable difference' values for even and uneven fading | | |
|---|---|---|
| Hue | JND for even fading / CIELAB $\Delta E$ | JND for uneven fading / CIELAB $\Delta E$ |
| Yellow | 1.50 | 1.00 |
| Magenta | 1.10 | 0.50 |
| Blue | 0.45 | 0.25 |
| Black | 0.75 | 0.35 |

## Example 3 - Correlation Studies:

A number of black cotton 'combat' style trousers were washed and dried for various numbers of cycles (between 1 and 10) using a number of different wash protocols (different detergent, drying method, ironing or not). The result of these various processes was to arrive at a wardrobe of clothes all showing different amounts of fading.

An area 10cm x 10cm on the leg of the garments was identified which contained a double stitched seam showing enhanced colour fading and an area of uniform 'background' colour fading. The reflectance spectrum of the background and the seam areas was measured using a Datacolor Spectraflash™ 500 spectrometer.

From these spectra, the CIELAB L* values were calculated for the seam and background areas of each garment. The garments were then presented, one at a time to each of 20 different panellists. The garments were presented in such a way that only the 10x10cm area was visible. The panellists were asked to score the level of fading on a 0 to 100 scale. The average score was calculated for each garment. From the data, two graphs were constructed: a plot of the L* value for the background area of the garment against the panel score; and a plot of the L* value of the seam against the panel score. The correlation coefficient was calculated for each plot.

These are tabulated below in Table 2. It is clear from these values that the lightness of the unevenly faded seam correlates more closely to the panellists' visual assessment of fading than does the lightness value of the evenly faded background fabric.

**Table 2:**

| Correlation between measured lightness and visual assessment | |
|---|---|
| | correlation coefficient |
| L* (background) vs panel score | 0.79 |

(continued)

| Correlation between measured lightness and visual assessment | |
|---|---|
| | correlation coefficient |
| L* (seam) vs panel score | 0.90 |

**Example 4 - Friction Measurements:**

The coefficient of friction between two pieces of white cotton fabric immersed in a solution of the lubricant material was measured using an Eldredge™ tribometer that had been modified to permit the study of submerged materials.

In summary, the set up consisted of a cylindrical watertight trough, to the bottom of which was affixed a 30 x 8 cm strip of plain, woven, white, non-mercerised 100% cotton sheeting. Above this was located a piece of similar fabric mounted a round a cylindrical holder 1 cm in diameter and 6 cm in length. This was attached to a pivoted arm which could be driven in such a way that the fabric cylinder was placed in contact with the stationery strip with defined normal load and then driven at a controlled velocity along a 6.5 cm length of the strip. Stain gauges on the arm permitted measurement of the frictional force opposing this motion. From the frictional force and the normal load it is possible to calculate the coefficient of friction ($\mu$). This measurement can be made for a range of velocities; but for present purposes it is appropriate to choose a sliding velocity of 6 cm s$^{-1}$.

For the purposes of assessing efficacy of potential lubricant systems, the index of wet cotton lubrication ($I_{wcl}$) is defined as:

$$I_{wcl} = \left( \frac{\mu_0 - \mu}{\mu_0} \right) \times 100$$

where $\mu_0$ is the coefficient of friction measured by the above method using a solution containing none of the lubrication.

**Examples 5-8: - formulations:**

The examples given below are typical formulations according to the present invention. The materials used in the examples are identified in table 3.

**Table 3:**

| Raw material specification | |
|---|---|
| Component | Specification |
| LAS | Linear Alkyl Benzene Sulphonic-acid, Marlon AS3™, ex Huls |
| Na-PAS | Primary Alkyl Benzene Sulphonic-acid, neutralised with NaOH |
| Dobanol™ 25-7 | $C_{12-15}$ ethoxylated alcohol, 7EO, ex Shell |
| Zeolite | Wassalith™ P, ex Degussa |
| STPP | Sodium Tri Polyphosphate, Thermphos™ NW, ex Hoechst |
| Dequest™ 2066 | Metal chelating agent, ex Monsanto |
| Lipolase | Type 100L, ex Novo |
| Savinase™ 16L | Protease, ex Novo |
| Sokalan™ CP5 | Acrylic/Maleic Builder Polymer, ex BASF |
| Deflocculating Polymer | Polymer A-11 disclosed in EP-A-346 995 |
| SCMC | Sodium Carboxymethyl Cellulose |

(continued)

| Raw material specification | |
|---|---|
| **Component** | **Specification** |
| Minors | Anti-redeposition polymers, transition-metal scavengers/bleach stabilisers, fluorescers, antifoam, dye-transfer-inhibition polymers, enzymes, perfume |

**Example 5 - Spray-Dried Powder:**

Example 5 provides a spray dried powder according to the present invention. The composition of the powder is given in Table 4.

**Table 4**

| Component | % w/w |
|---|---|
| Na PAS | 11.5 |
| Dobanol 25-7 | 6.3 |
| Soap | 2 |
| Zeolite | 24.1 |
| SCMC | 0.6 |
| Na Citrate | 10.6 |
| Na Carbonate | 23 |
| sodium polyacrylate (mol wt 1 300 000) | 0.7 |
| Dequest 2066 | 0.4 |
| Sokalan CP5 | 0.9 |
| Savinase 16L | 0.7 |
| Lipolase | 0.1 |
| Perfume | 0.4 |
| Water/salts | Up to 100% |

**Example 6 - Detergent granulate prepared by Non-Spray Drying Method:**

Example 6 provides the formulation of a granulate manufactured according to the present invention by a non-tower route.

**Table 5**

| Component | % w/w |
|---|---|
| Na PAS | 13.5 |
| Dobanol 25-7 | 2.5 |
| STPP | 45.3 |
| Na Carbonate | 4 |
| Polyacrylamide (mol wt 5-6 000 000) | 2.8 |
| Na Silicate | 10.1 |
| Minors | 1.5 |
| Water | Up to 100% |

### Example 7 - Isotropic Laundry Liquid:

Example 7 provides an example of an isotropic laundry liquid according to the present invention. The formulation is given in Table 6:

**Table 6**

| Component | % w/w |
|---|---|
| Na Citrate | 10.7 |
| Propylene Glycol | 7.5 |
| Ethylene Glycol | 4.5 |
| Borax | 3 |
| Savinase 16L | 0.3 |
| Lipolase | 0.1 |
| Dextran (mol wt 800 000) | 1.0 |
| Monoethanolamine | 0.5 |
| Cocofatty acid | 1.7 |
| NaOH (50%) | 2.2 |
| LAS | 10.3 |
| Dobanol 25-7 | 6.3 |
| LES | 7.6 |
| Minors (adjust pH to 7 white NaOH) | 1.3 |
| Water | Up to 100% |

### Example 8, Structured Laundry Liquids

Example 8 provides an example of a structured laundry.liquid according to the present invention. The formulation of the product is given in Table 7.

**Table 7**

| Component | % w/w |
|---|---|
| LAS | 16.5 |
| Dobanol 25-7 | 9 |
| Oleic acid (Priolene (6907)) | 4.5 |
| Zeolite | 15 |
| KOH, neutralisation of acids and pH to | 8.5 |
| Citric acid | 8.2 |
| Deflocculating Polymer | 1 |
| Protease | 0.38 |
| Lipolase | 0.2 |
| Imacol C (ex Clariant) | 2.0 |
| Minors | 0.4 |
| Water | Up to 100% |

9

**Example 9 - Powder Formulation:**

Example 9 provides an example of a further powder formulation. The composition of which is given in Table 8.

**Table 8**

| Component | % w/w |
|---|---|
| Na LAS | 6.3 |
| Nonionic 3EO branched | 3.15 |
| Nonionic 7EO branched | 4.05 |
| Soap | 0.28 |
| sodium tripolyphosphate | 23.9 |
| Sodium perborate | 20.00 |
| Sodiumsilicate | 6.31 |
| Tetra acetyl ethylene diamine | 2.22 |
| SCMC | 0.23 |
| Sodium sulphate | 9.87 |
| Na Carbonate | 8.49 |
| sodium polyacrylate (mol wt 1 300 000) | 5.0 |
| Dequest 2047 | 0.55 |
| Sokalan CP5 | 1.13 |
| Savinase 12 TXT | 0.40 |
| Lipolase | 0.1 |
| Perfume | 0.24 |
| Water/salts | Up to 100% |

**Claims**

1. Use of an anionic or nonionic polymeric lubricant, which does not adsorb onto textiles, in a main wash composition, during the laundering process, to prevent the visible appearance of local colour loss through mechanical damage by the laundering process.

2. Use according to claim 1, wherein the lubricant has an index of wet cotton lubrication greater than 15 when measured with a solution containing 1 g dm$^{-3}$ of the lubricant, wherein the index of wet cotton lubrication is defined as:

$$I_{wcl} = \left( \frac{\mu_0 - \mu}{\mu_0} \right) \times 100$$

where $\mu_0$ is the coefficient of friction measured using a solution containing none of the lubrication.

3. Use according to claim 1, wherein the lubricant is a material which when dissolved at a concentration of 1 g dm$^{-3}$ in water at 25 °C gives a solution that has a viscosity greater than 0.05 Pa s when measured at a shear rate of 100 s$^{-1}$.

4. Use according to claim 1 wherein the lubricant has a molecular weight greater than 100,000 Dalton.

5. Use according to claim 4 wherein the lubricant is a polyacrylate, polyacrylic acid, polyacrylamide, poly vinyl pyrrolidone or a co-polymer thereof.

6. Use according to claim 1 wherein the lubricant is a polydimethyl siloxane.

7. Use according to claim 1 wherein the lubricant is an oxidised polyethylene wax.

8. Use according to claim 1 wherein the lubricant is present at a level of 0-5-5%wt.

9. Use according to claim 1 wherein the articles being laundered are garments.

**Patentansprüche**

1. Verwendung eines anionischen oder nicht-ionischen polymeren Schmiermittels, das nicht auf Textilien adsorbiert, in einer Hauptwäschezusammensetzung während des Wäschewaschverfahrens, um so das sichtbare Auftreten eines lokalen Farbverlustes durch mechanische Schädigung in dem Wäschewaschverfahren zu verhindern.

2. Verwendung nach Anspruch 1, worin das Schmiermittel einen Schmierungsindex für nasse Baumwolle von größer als 15 hat, wenn er mit einer Lösung, die 1 g dm$^{-3}$ Schmiermittel enthält, gemessen wird, wobei der Schmierungsindex für nasse Baumwolle wie folgt definiert wird:

$$I_{wcl} = \left( \frac{\mu_0 - \mu}{\mu_0} \right) \times 100$$

,

worin $\mu_0$ der Reibungskoeffizient, gemessen unter Verwendung einer Lösung, die kein Schmiermittel enthält, ist.

3. Verwendung nach Anspruch 1, worin das Schmiermittel ein Material ist, das, wenn es bei einer Konzentration von 1 g dm$^{-3}$ in Wasser bei 25 °C gelöst wird, eine Lösung ergibt, die eine Viskosität größer als 0,05 Pa s hat, gemessen bei einer Scherrate von 100 s$^{-1}$.

4. Verwendung nach Anspruch 1, worin das Schmiermittel ein Molekulargewicht von mehr als 100.000 Dalton hat.

5. Verwendung nach Anspruch 4, worin das Schmiermittel ein Polyacrylat, eine Polyacrylsäure, ein Polyacrylamid, ein Polyvinylpyrrolidon oder ein Copolymer davon ist.

6. Verwendung nach Anspruch 1, worin das Schmiermittel ein Polydimethylsiloxan ist.

7. Verwendung nach Anspruch 1, worin das Schmiermittel ein oxidiertes Polyethylenwachs ist.

8. Verwendung nach Anspruch 1, worin das Schmiermittel bei einem Niveau 0,5 - 5 Gew.-% vorliegt.

9. Verwendung nach Anspruch 1, worin die zu waschenden Artikel Kleidungsstücke sind.

**Revendications**

1. Utilisation d'un lubrifiant polymérique anionique ou non ionique qui ne s'adsorbe pas sur les textiles, dans une composition de lavage principal pendant le processus de lessivage, pour prévenir l'apparition visible d'une perte locale de couleur due aux dommages mécaniques pendant le processus de lessivage.

2. Utilisation selon la revendication 1, dans laquelle le lubrifiant a un taux d'ensimage sur du coton mouillé supérieur à 15 lorsqu'on le mesure avec une solution contenant 1 g dm$^{-3}$ du lubrifiant, où le taux d'ensimage sur du coton mouillé se définit comme :

$$I_{wcl} = \left( \frac{\mu_0 - \mu}{\mu_0} \right) \times 100$$

dans laquelle $\mu_0$ est le coefficient de frottement mesuré en utilisant une solution ne contenant pas de lubrification.

3. Utilisation selon la revendication 1, dans laquelle le lubrifiant est un matériau qui, lorsqu'il est dissout à une concentration de 1 g dm$^{-3}$ dans de l'eau à 25°C, donne une solution ayant une viscosité supérieure à 0,05 Pa.s lorsqu'on la mesure à un taux de cisaillement de 100 s$^{-1}$.

4. Utilisation selon la revendication 1, dans laquelle le lubrifiant a une masse moléculaire supérieure à 100 000 Dalton.

5. Utilisation selon la revendication 4, dans laquelle le lubrifiant est un polyacrylate, un acide polyacrylique, un polyacrylamide, une polyvinylpyrrolidone ou un copolymère de ceux-ci.

6. Utilisation selon la revendication 1, dans laquelle le lubrifiant est un polydiméthyle siloxane.

7. Utilisation selon la revendication 1, dans laquelle le lubrifiant est une cire de polyéthylène oxydée.

8. Utilisation selon la revendication 1, dans laquelle le lubrifiant est présent à un niveau de 0,5 - 5 % en poids.

9. Utilisation selon la revendication 1, dans laquelle les articles lessivésont des vêtements.